# EUROPÄISCHE PATENTANMELDUNG

(11) **EP 4 837 524 A2**
(43) Veröffentlichungstag der Anmeldung: **11.11.2026**
(21) Anmeldenummer: 26194928.3
(22) Anmeldetag: 23.05.2022
(51) Int. Cl.: A61B 17/16

(54) **IMPLANTATBOHRER, INSBESONDERE DENTALIMPLANTATBOHRER**

(30) Priorität: 05.07.2021 DE 102021117272
(62) Dokumentnummer(n) der früheren Anmeldung(en) nach Art. 76 EPÜ: 24208538.9 / 4 520 276; 22731107.3 / 4 355 258
(71) Anmelder: medentis medical GmbH, 53474 Bad Neuenahr-Ahrweiler (DE)
(72) Erfinder: Scholz, Alexander, 53474 Bad Neuenahr-Ahrweiler (DE)
(74) Vertreter: Müller Schupfner & Partner Patent- und Rechtsanwaltspartnerschaft mbB (Muc)

(57) **Zusammenfassung**

Implantatbohrer (1), insbesondere Dentalimplantatbohrer, umfassend einen Montagebereich (10) und einen Bearbeitungsbereich (30), wobei sich der Implantatbohrer (1) in eine Längsrichtung (L) erstreckt, wobei die distalen Endbereiche in Längsrichtung (L) des Implantatbohrers (1) insbesondere durch den Montagebereich (10) und den Bearbeitungsbereich (30) ausgebildet sind, wobei der Bearbeitungsbereich (30) zumindest zwei Bearbeitungsstrukturen (32) aufweist, wobei der Bearbeitungsbereich (30), insbesondere durch die Bearbeitungsstrukturen (32), derart ausgebildet ist, dass dieser bei einer positiven Rotation um die Längsrichtung (L) Knochenmaterial abtragen kann und dass dieser bei einer negativen Rotation um die Längsrichtung (L) Knochenmaterial verdichten kann.

## Beschreibung

Die Erfindung betrifft einen Implantatbohrer, insbesondere ein Dentalimplantatbohrer.

Implantate sind bereits aus dem Stand der Technik bekannt. Diese tragen meist eine Prothese und sind in dem umgebenden Knochen fest verankert. Um ein Implantat in den Knochen einzusetzen, muss dabei eine Ausnehmung in dem Knochen geschaffen werden. In Abhängigkeit des umgebenden Knochens muss dabei jeweils entschieden werden, ob die Ausnehmung in den Knochen durch ein Bohren oder durch ein Verdichten geschaffen wird. Diese beiden Tätigkeiten werden dabei im Stand der Technik durch unterschiedliche Werkzeuge erreicht, nämlich zum einen durch einen Bohrer bzw. einen Satz von Bohrern und zum anderen durch ein oder eine Vielzahl von Verdichtungswerkzeugen, wie z.B. einem Meißel. Die Bereitstellung verschiedener Werkzeuge kann dabei zu Verwechslungen führen sowie auch zu einem hohen Arbeitsaufwand.

Die US 2021/196286 A1 betrifft einen Bohrer, welcher in einer Drehrichtung verdichtet und in der anderen Drehrichtung schneidet.

Die US 2005/118 550 A1, die WO 2018/202605 A1, die DE 10 2005 011 917 A1, die EP 0 515 274 A1 und die WO 2021 020 656 A1 betreffen ebenfalls Implantatbohrer.

Es ist daher die Aufgabe der vorliegenden Erfindung, eine einfache und zuverlässige Möglichkeit zu schaffen, eine Ausnehmung für ein Implantat in einem Knochen, wie einem Kieferknochen, insbesondere eines Menschens, bereitstellen zu können.

Diese Aufgabe kann mit einem Implantatbohrer gemäß dem Ausführungsbeispiel 1 gelöst werden. Weitere Merkmale, Vorteile und Ausführungsformen ergeben sich aus den abhängigen Ausführungsbeispielen, der Beschreibung sowie aus den Figuren.

Erfindungsgemäß kann ein Implantatbohrer, insbesondere ein Dentalimplantatbohrer vorgesehen sein. Zweckmäßigerweise umfasst der Implantatbohrer einen Montagebereich und einen Bearbeitungsbereich, wobei sich der Implantatbohrer, insbesondere in eine bzw. entlang einer Längsrichtung erstreckt, wobei die distalen Endbereiche in Längsrichtung des Implantatbohrers insbesondere durch den Montagebereich und den Bearbeitungsbereich ausgebildet sind, wobei der Bearbeitungsbereich zumindest zwei Bearbeitungsstrukturen aufweisen kann, wobei der Bearbeitungsbereich, insbesondere durch die Bearbeitungsstrukturen, derart ausgebildet ist, dass dieser bei einer positiven Rotation um die Längsrichtung Knochenmaterial abtragen kann und dass dieser bei einer negativen Rotation um die Längsrichtung Knochenmaterial verdichten kann. Der Implantatbohrer dient dazu, durch eine rotatorische Bewegung eine Ausnehmung bzw. ein Loch in einem Knochen, insbesondere einem Kiefer eines Menschen, bereitstellen zu können. Dieses Loch dient dazu, ein Implantat, insbesondere ein Dentalimplantat, aufnehmen zu können. Der Implantatbohrer umfasst einen Montagebereich, welcher dazu ausgelegt ist bzw. dazu dient, den Implantatbohrer in einer Werkzeugmaschine bzw. einer Bearbeitungsmaschine bzw. einem Handbearbeitungsgerät festzulegen. Diese Festlegung ist insbesondere derart, dass eine rotatorische Bewegung, insbesondere formschlüssig, auf den Implantatbohrer übertragen werden kann und/oder dass der Implantatbohrer in Richtung der Längsrichtung, insbesondere formschlüssig, gehalten werden kann. Zweckmäßigerweise weist der Montagebereich des Implantatbohrers hierzu formschlüssige Strukturen auf, welche es erlauben, formschlüssig ein Drehmoment um die Längsrichtung und/oder eine Haltekraft entlang der Längsrichtung übertragen zu können. Beispielsweise kann dies durch eine umlaufende Nut und/oder durch eine Sektionalfläche erreicht werden, welche z.B. auch als Abflachung bezeichnet werden kann. Neben dem Montagebereich verfügt der Implantatbohrer auch über einen Bearbeitungsbereich. Dieser Bearbeitungsbereich ist insbesondere derjenige Bereich des Implantatbohrers, welcher während einer Bearbeitung in unmittelbaren Kontakt mit dem Knochenmaterial tritt, um so die Ausnehmung bzw. das Loch im Kiefer bzw. im Knochenmaterial zu erreichen bzw. zu erstellen. In anderen Worten dient der Bearbeitungsbereich des Implantatbohrers dazu, das Loch zu kreieren. Der Implantatbohrer erstreckt sich insbesondere in eine Längsrichtung, wobei die Längsrichtung zweckmäßigerweise diejenige Richtung ist, in welche der Implantatbohrer bei der Bearbeitung rotiert bzw. um welche der Implantatbohrer rotiert. Alternativ oder zusätzlich bevorzugt kann die Längsrichtung auch diejenige Richtung sein, in welche der Implantatbohrer seine größte Hauptabmessung aufweist. Zweckmäßigerweise ist einer der distalen Endbereiche in Längsrichtung des Implantatbohrers durch den Montagebereich und/oder, insbesondere gegenüberliegend, durch den Bearbeitungsbereich ausgebildet. Vorteilhafterweise weist dabei die positive Längsrichtung von dem Montagebereich zum Bearbeitungsbereich oder umgekehrt. Durch die Ausbildung des Bearbeitungsbereichs als distaler Endbereich des Implantatbohrers in Längsrichtung kann eine besonders einfache Bearbeitungsmöglichkeit durch den Implantatbohrer geschaffen werden. Durch das Ausbilden eines, insbesondere gegenüberliegenden, distalen Endbereichs in Längsrichtung durch den Montagebereich, kann eine besonders einfache Montage des Implantatbohrers in einer Werkzeugaufnahme, insbesondere einer Bohrmaschine, einem Fräser oder einer Werkzeugmaschine oder einem Handwerkzeug, erreicht werden. Der Bearbeitungsbereich des Implantatbohrers verfügt vorteilhafterweise über zumindest zwei Bearbeitungsstrukturen. Diese Bearbeitungsstrukturen sind insbesondere diejenigen Flächen, Kanten oder anderweitige Strukturen des Bearbeitungsbereichs, welche eine Knochenumformung und/oder eine Knochenabtrennung bewirken, insbesondere wenn der Bohrer in Rotationversetzt wird. In anderen Worten können die flächigen oder linienförmigen Bearbeitungsstrukturen eine Umformung und/oder einen Abtrag von Knochenmaterial bewirken, wobei diese Bearbeitung bzw. Formänderung durch die Rotation des Implantatbohrers um die Längsrichtung erreicht werden kann bzw. wird. Der Bearbeitungsbereich ist, insbesondere durch die Bearbeitungsstrukturen, derart ausgebildet, dass dieser bei einer positiven Rotation um die Längsrichtung Knochenmaterial abtragen kann und dass dieser bei einer negativen Rotation um die Längsrichtung Knochenmaterial verdichten kann. In anderen Worten kann der Bearbeitungsbereich des Implantatbohrers derart ausgebildet sein, dass bei einer Rotation des Implantatbohrers um die Längsrichtung in die eine Richtung ein Abtragen von Knochenmaterial stattfindet, wie es beispielsweise auch bei einem Fräser oder Bohrer der Fall ist. Sollte der Bohrer jedoch in die entgegengesetzte Richtung um die Längsrichtung rotieren, so ist der Bearbeitungsbereich derart ausgebildet, dass dieser eine Verdichtung des zu bearbeitenden Knochenmaterials, insbesondere durch einen unmittelbaren Kontakt geneigter Flächen mit dem Knochenmaterial, bewirkt. Durch diese Ausgestaltung der unterschiedlichen Bearbeitungsmöglichkeiten des Implantatbohrers ermöglicht dieser, dass durch ein einziges Werkzeug sowohl eine Abtragung von Knochenmaterial als auch eine Verdichtung des Knochenmaterials möglich ist. Zweckmäßigerweise ist der Implantatbohrer bzw. der Bearbeitungsbereich, insbesondere die Bearbeitungsstrukturen, derart ausgebildet, dass diese(r) bei einer positiven Rotation um die Längsrichtung ausschließlich Knochenmaterial abtragen kann und dass dieser bei einer negativen Rotation um die Längsrichtung ausschließlich Knochenmaterial verdichten kann. In anderen Worten kann der Implantatbohrer derart ausgebildet sein, dass bei einer Rotation um die Längsrichtung, beispielsweise bei einer positiven Rotation um die Längsrichtung, ausschließlich Knochenmaterial abgetragen wird, insbesondere durch einen ausschließlichen Kontakt von geometrisch bestimmten Schneiden mit dem Knochenmaterial, und dass bei einer entgegengesetzten Rotation um die Längsrichtung ausschließlich knochenverdichtende Bearbeitungsstrukturen mit dem Knochen in Kontakt gelangen, so dass bei einer derartigen Rotation eine ausschließliche Knochenverdichtung stattfindet.

Vorteilhafterweise weist der Bearbeitungsbereich zumindest zwei, bevorzugt zumindest drei, Spiralnuten auf, wobei die Spiralnuten zumindest teilweise die Bearbeitungsstrukturen oder einige Bearbeitungsstrukturen ausbilden und/oder begrenzen. Unter einer Spiralnut ist dabei eine Nut zu verstehen, welche spiralförmig um die Längsrichtung in den Implantatbohrer eingebracht ist. Durch die Einbringung einer Spiralnut kann in besonders kompakter Weise eine Ausgestaltung der Bearbeitungsstruktur, insbesondere von Bohrbearbeitungsstrukturen, erreicht werden. Darüber hinaus können die Spiralnuten auch in einfacher Weise dazu genutzt werden, einen Aufnahmeraum für abgetragenes Knochenmaterial bereitzustellen. Durch das Vorsehen von zumindest zwei, bevorzugt von zumindest drei Spiralnuten, kann eine hohe Kühlungswirkung erreicht werden und darüber hinaus kann durch diese Art der Ausgestaltung auch eine besonders homogene Eingriffscharakteristik des Bearbeitungsbereichs mit einem Knochenmaterial während der Bearbeitung erreicht werden, so dass hierdurch die auftretenden Vibrationen während der Bearbeitung des Knochenmaterials durch den Implantatbohrer reduziert werden können.

Zweckmäßigerweise bildet die Spiralnut zumindest teilweise die Bearbeitungsstrukturen oder einige Bearbeitungsstrukturen aus und/oder begrenzt diese, insbesondere in eine Umfangsrichtung. Diese Umfangsrichtung ist insbesondere umlaufend um die Längsrichtung ausgebildet. Durch das Ausnutzen der Spiralnuten dahingehend, dass diese teilweise die Bearbeitungsstrukturen ausbilden und/oder begrenzen, kann eine besonders kompakte Ausgestaltung des Implantatbohrers erreicht werden.

Vorteilhafterweise weist der Bearbeitungsbereich entlang der Längsrichtung unterschiedliche Bearbeitungsabschnitte auf, wobei der Implantatbohrer vorzugsweise zumindest drei Bearbeitungsabschnitte aufweist, wobei die Bearbeitungsabschnitte unterschiedliche Außenkontursteigungswinkel mit der Längsrichtung ausbilden oder zumindest einer der Bearbeitungsabschnitte, vorzugsweise alle, zylindrisch um die Längsrichtung ausgebildet sind. Unter einem Außenkontursteigungswinkel ist dabei derjenige Winkel zu verstehen, welcher ein Konus aufweist, der gerade den Bearbeitungsabschnitt umgeben kann und wobei die Rotationssymmetrieachse dieses Konuses auf der Längsrichtung liegt. Vorteilhafterweise ist dabei der Außenkontursteigungswinkel konstant über den jeweiligen Bearbeitungsabschnitt. Hierdurch kann eine besonders einfache Bearbeitung bzw. Herstellung des jeweiligen Bearbeitungsabschnitts erreicht werden. Unter einer zylindrischen Ausgestaltung um die Längsrichtung der Bearbeitungsabschnitte ist dabei insbesondere zu verstehen, dass die Außenkontur des Konuses, welcher gerade den Bearbeitungsabschnitt umgibt und dessen Rotationssymmetrieachse auf der Längsrichtung liegt, die Längsrichtung selber erst im Unendlichen schneiden kann. In anderen Worten ist die Außenkontur nicht durch einen Konus sondern durch einen Zylinder umschließbar bzw. umschlossen. Der Vorteil einer zylindrischen Ausgestaltung liegt dabei darin, dass hierdurch eine besonders einfache Herstellung erreicht werden kann. Der Vorteil der verschiedenen Winkel der verschiedenen Bearbeitungsabschnitte, welche auch als Bearbeitungssektionen bezeichnet werden können, liegt darin, dass hierdurch ermöglicht wird, dass durch den Bohrer ein werkzeugwechselfreies Erstellen der Implantatausnehmung im Knochenmaterial erreicht werden kann. Insbesondere durch die verschiedenen Bearbeitungssektionen bzw. Bearbeitungsabschnitte kann daher der Implantatbohrer derart ausgebildet sein, dass bis auf ein mögliches Vorbohren, die Ausnehmung im Knochen für das Implantat ausschließlich mit dem einen Implantatbohrer erfolgen kann. Daher können zeitaufwendige Werkzeugwechsel eingespart werden und eine Verwechslung von Werkzeugen kann verhindert oder zumindest reduziert werden. Zweckmäßigerweise ist der Implantatbohrer derart ausgestaltet, dass Bearbeitungsabschnitte mit Außenkontursteigungswinkel und Bearbeitungsabschnitte mit zylindrischer Ausgestaltung alternierend miteinander in Längsrichtung angeordnet sind. Zweckmäßigerweise ist dabei jeder Bearbeitungsabschnitt (für sich selbst) derart ausgebildet, dass diese bei einer positiven Rotation um die Längsrichtung Knochenmaterial abtragen kann und dass diese bei einer negativen Rotation um die Längsrichtung Knochenmaterial verdichten kann. In anderen Worten können die Bearbeitungsabschnitte sich ausschließlich im Hinblick auf ihre geometrische Ausgestaltung voneinander unterscheiden, jedoch in ihrer Funktionsweise äquivalent ausgebildet sein.

Vorteilhafterweise ist der Implantatbohrer, insbesondere der Bearbeitungsbereich, durch einen Axialbearbeitungsabschnitt in Längsrichtung begrenzt, wobei der Axialbearbeitungsabschnitt einen Außenkontursteigungswinkel mit der Längsrichtung ausbildet, wobei der Außenkontursteigungswinkel des Axialbearbeitungsabschnitts in einem Bereich von 60° bis 87° liegt und/oder wobei die Länge des Axialbearbeitungsabschnitts in Längsrichtung in einem Bereich von 0,2 mm bis 0,4 mm liegt. Durch das Vorsehen eines Axialbearbeitungsabschnitts, dessen Kernaufgabe das axiale Eindringen in ein Knochenmaterial ist, kann eine besonders gute Möglichkeit des Eindringens in den Kieferknochen erreicht werden, wenn dieser den Bearbeitungsbereich in Längsrichtung begrenzt. Sollte der Axialbearbeitungsabschnitt einen Außenkontursteigungswinkel mit der Längsrichtung ausbilden, welcher in einem Bereich von 60° bis 87° liegt, kann hierdurch erreicht werden, dass eine besonders schnelle und dennoch effektive axiale Eindringungsmöglichkeit des Implantatbohrers in das Knochenmaterial bereitgestellt werden kann. Sollte die Länge des Axialbearbeitungsabschnitts in Längsrichtung in einem Bereich von 0,2 mm bis 0,4 mm liegen, so kann hierdurch eine besonders kompakte Ausgestaltung des Axialbearbeitungsabschnitts erreicht werden, so dass ein besonders einfach zu handhabender Implantatbohrer erreicht werden kann, wobei dies insbesondere bei Dentalimplantatbohrern entscheidend sein kann, denn gerade im Dentalbereich ist der Handhabungsbereich für einen Bohrer besonders gering.

Zweckmäßigerweise weist der Implantatbohrer, insbesondere der Bearbeitungsbereich, einen Hauptbearbeitungsabschnitt auf, wobei der Hauptbearbeitungsabschnitt einen Außenkontursteigungswinkel mit der Längsrichtung ausbildet, wobei der Außenkontursteigungswinkel des Hauptbearbeitungsabschnitts in einem Bereich von 0,2° bis 9° liegt, oder wobei der Hauptbearbeitungsabschnitt zylindrisch um die Längsrichtung ausgebildet ist. Der Hauptbearbeitungsabschnitt ist insbesondere der Bearbeitungsabschnitt des Implantatbohrers, welcher die höchste bzw. größte Länge in Längsrichtung aufweist. Der Hauptbearbeitungsabschnitt dient dabei vornehmlich dazu, eine endgültige Ausgestaltung des Implantataufnahmelochs bzw. der Knochenausnehmung, welche durch den Implantatbohrer geschaffen werden soll, sicher zu stellen. Insbesondere bildet der Hauptbearbeitungsabschnitt daher bevorzugt ein distales Ende der Knochenausnehmung aus. Sollte der Hauptbearbeitungsabschnitt einen Außenkontursteigungswinkel mit der Längsrichtung ausbilden, wobei dieser bevorzugt in einem Bereich von 0,2° bis 9° liegen kann, so kann hierdurch eine besonders einfache und gute Montierbarkeit, insbesondere von selbstschneidenden Implantaten, zusätzlich oder alternativ bevorzugt Dentalimplantaten, erreicht werden. Sollte der Hauptbearbeitungsabschnitt jedoch zylindrisch um die Längsrichtung ausgebildet sein, so kann hierdurch eine besonders einfache Herstellung des Bohrers erreicht werden und darüber hinaus eine gute Haltekraftentwicklung für das Implantat im Knochen erzielt werden.

Vorteilhafterweise weist der Implantatbohrer, insbesondere der Bearbeitungsbereich, besonders bevorzugt der Hauptbearbeitungsabschnitt, umlaufende Kühlnuten auf, wobei die Kühlnuten insbesondere geschlossene Ringe um die Längsrichtung ausbilden. Durch das Vorsehen von Nuten bzw. Kühlnuten wird zum einen die zur Verfügung stehende Oberfläche vergrößert und zum anderen kann hierdurch auch die Schneidfläche der schneidenden Bearbeitungsstrukturen, insbesondere der Bohrbearbeitungsstrukturen, verkleinert werden, wobei beide diese Effekte zur Kühlung beitragen können. Das Bereitstellen einer ausreichenden Kühlung ist dabei grundlegend wichtig, um ein überhitzungsbedingtes Trauma des zu bearbeitenden Knochenmaterials zu verhindern. Die Kühlnuten verlaufen dabei insbesondere in geschlossenen Ringen um die Längsrichtung. Unter einem geschlossenen Ring ist dabei zu verstehen, dass die Kühlnuten bzw. die gedachte Verlängerung der Kühlnuten in sich selber geschlossen ist und vorteilhafterweise kreisförmig ausgebildet ist. In anderen Worten kann daher die Kühlnut entlang ihres Verlaufs beispielsweise durch Spiralnuten oder durch Bearbeitungsstrukturen unterbrochen sein, wobei jedoch hinter dieser Unterbrechung die Kühlnut fortgesetzt wird und zumindest der Kühlnutverlauf in Komplementierung mit dem gedachten Verlauf eine in sich geschlossene Struktur, insbesondere eine kreisförmige in sich geschlossene Struktur, ausbildet. Durch die geschlossenen Ringe der Kühlnut kann eine besonders einfache und schnelle Fertigung erreicht werden. Zweckmäßigerweise liegt dabei die Erstreckungsebene dieser geschlossenen Ringe senkrecht zur Längsrichtung. Hierdurch kann die Fertigung der Kühlnuten weiter vereinfacht werden. Alternativ oder zusätzlich bevorzugt kann der Mittelpunkt, um welchen die Kühlnut oder die Kühlnuten ausgebildet sind, auf der Längsrichtung liegen, um eine schnelle und einfache sowie präzise Fertigung zu erreichen.

Zweckmäßigerweise weist der Implantatbohrer, insbesondere der Bearbeitungsbereich, einen Zylinderbearbeitungsabschnitt auf, wobei der Zylinderbearbeitungsabschnitt zylindrisch um die Längsrichtung ausgebildet ist. Zweckmäßigerweise ist der Zylinderbearbeitungsabschnitt dabei in Längsrichtung zwischen dem Axialbearbeitungsabschnitt und dem Hauptbearbeitungsabschnitt ausgebildet. Besonders zweckmäßig ist es dabei, wenn der Zylinderbearbeitungsabschnitt in Längsrichtung benachbart zum Axialbearbeitungsabschnitt ausgebildet ist. Durch die zylindrische Ausgestaltung des Zylinderbearbeitungsabschnitts kann eine besonders gute radiale Führung des Bohrers erreicht werden. Diese Führungswirkung des Zylinderbearbeitungsabschnitts kann dadurch gesteigert werden, dass der Zylinderbearbeitungsabschnitt in Längsrichtung direkt anschließend zu dem Axialbearbeitungsabschnitt angeordnet ist und/oder zumindest in Längsrichtung zwischen dem Axialbearbeitungsabschnitt und dem Hauptbearbeitungsabschnitt.

Bevorzugt weist der Implantatbohrer, insbesondere der Bearbeitungsbereich, einen Übergangsbearbeitungsabschnitt auf, wobei der Übergangsbearbeitungsabschnitt einen Außenkontursteigungswinkel mit der Längsrichtung ausbildet, wobei der Außenkontursteigungswinkel des Übertragungsabschnitts insbesondere in einem Bereich von 7° bis 15° liegt. Durch das Vorsehen eines Übergangsbearbeitungsabschnitts kann in besonders einfacher Weise eine einfach herzustellende Durchmessersteigerung des Bearbeitungsbereichs erreicht werden. Der Übergangsbearbeitungsabschnitt befindet sich in Längsrichtung, insbesondere zwischen dem Axilabearbeitungsabschnitt und dem Hauptbearbeitungsabschnitt. Zweckmäßigerweise bildet der Übergangsbearbeitungsabschnitt dabei denjenigen Abschnitt des Bearbeitungsbereichs aus, welcher in Längsrichtung zwischen dem Zylinderbearbeitungsabschnitt und dem Hauptbearbeitungsabschnitt ausgebildet ist. Durch das Ausbilden des Übergangsbearbeitungsabschnitts mit einem Außenkontursteigungswinkel in einem Bereich von 7° bis 15° kann eine besonders geringe Wärmeentwicklung während der Bearbeitung des Knochenmaterials erreicht werden, so dass hierdurch überhitzungsbedingte Knochentraumata vermieden oder zumindest reduziert werden können.

Vorteilhafterweise liegt das Verhältnis des Durchmessers des Zylinderbearbeitungsabschnitts zu dem Durchmesser des Hauptbearbeitungsabschnitts in einem Bereich von 0,5 bis 0,9, bevorzugt in einem Bereich von 0,72 bis 0,82. Der Durchmesser des Zylinderbearbeitungsabschnitts ist dabei der Durchmesser der den Zylinderbearbeitungsabschnitt gerade umgebenden Zylinders, wobei dieser gedachte Zylinder eine Rotationssymmetrieachse aufweist, welche deckungsgleich mit der Längsrichtung ist. In anderen Worten ist der Durchmesser des Zylinderbearbeitungsabschnitts dabei der Durchmesser desjenigen Kreises, welcher den Zylinderbearbeitungsabschnitt bzw. den Hauptbearbeitungsabschnitt in einer Schnittebene senkrecht zur Längsrichtung gerade umgeben kann und wobei der Mittelpunkt dieses Kreises auf der Längsrichtung liegt. Dieser kleinstmögliche Kreis in der Schnittebene wird dabei im Sinne der Erfindung insbesondere auch als eine Umhüllende bezeichnet. Bei einem Verhältnis im Bereich von 0,5 bis 0,9 kann eine besonders einfache Herstellung des Implantatbohrers erreicht werden. Sollte das Verhältnis jedoch im Bereich von 0,72 bis 0,82 liegen, so kann hierdurch eine besonders gute Führungswirkung durch den Zylinderbearbeitungsabschnitt erreicht werden.

Zweckmäßigerweise liegt das Verhältnis der Länge des Zylinderbearbeitungsabschnitts in Längsrichtung zu der Länge des Hauptbearbeitungsabschnitts in Längsrichtung in einem Bereich von 0,1 bis 0,4, bevorzugt in einem Bereich von 0,2 bis 0,3. Bei einem Verhältnis im Bereich von 0,1 bis 0,4 kann eine besonders gute Führungswirkung des Zylinderbearbeitungsabschnitts - auch bei einer Verdichtung des Knochenmaterials - bereitgestellt werden. Sollte jedoch das Verhältnis in einem Bereich von 0,2 bis 0,3 liegen, so hat die Anmelderin überraschend herausgefunden, dass hierdurch eine Ausnehmung geschaffen werden kann, welche eine besonders gute Haltbarkeit eines Implantats erreichen kann.

Vorteilhafterweise weist der Bearbeitungsbereich zumindest eine Verdichtungsbearbeitungsstruktur und eine Bohrbearbeitungsstruktur auf. Unter einer Bohrbearbeitungsstruktur ist inbesondere eine Bearbeitungsstruktur zu verstehen, welche eine geometrisch bestimmte Schneide aufweist. Eine geometrisch bestimmte Schneide dient dabei insbesondere dazu, durch Spanabheben eine Bearbeitung eines Knochenmaterials zu erreichen. Eine derartige Bohrbearbeitungsstruktur kann beispielsweise durch eine Hinterschneidung von zwei miteinander in Kontakt tretenden (Grenz-)Flächen erreicht werden. In anderen Worten kann die Bohrbearbeitungsstruktur insbesondere die Schnittkante von zwei ineinander übergehenden Flächen sein, wobei diese Flächen insbesondere eine Hinterschneidung miteinander ausbilden können. Eine Verdichtungsbearbeitungsstruktur ist insbesondere eine zur Bearbeitung von Knochen vorgesehene Struktur, welche beispielsweise durch eine nachlaufende Fläche gebildet sein kann, die einen Druck auf den Knochen ausüben kann, um diesen, insbesondere in Normalenrichtung zu der Wand des zu kreierenden Lochs, zu verdrängen, und so eine Verdichtung des Knochenmaterials erreichen. Beispielsweise kann eine derartige Verdichtungsbearbeitungsstruktur durch eine Freiformfläche bzw. eine Freifläche erreicht werden, welche derart ausgebildet ist, dass diese durch die Rotation eine Knochenverdichtung bewirkt, bzw. lokal ein an der Fläche anliegendes Knochenmaterial nach außen schiebt bzw. verdrängt. Dies kann beispielsweise dadurch erreicht werden, dass die Freiformfläche bzw. die Freifläche einen Winkel ausbildet und dadurch das Knochenmaterial nach außen verdrängt. Insbesondere kann diese Freiformfläche bzw. Freifläche derart ausgebildet sein, dass diese eine in Umlaufrichtung ansteigende Fläche ist, welche insbesondere bei einer negativen Rotation um die Längsrichtung derart ausgebildet ist, dass die Freifläche einen zunehmenden Abstand zur Rotationsachse bzw. zur Längsrichtung ausbildet. In anderen Worten kann daher diese Freifläche bzw. Freiformfläche hinterschneidungsfrei ausgebildet sein, so dass diese bei einer Rotation, insbesondere bei einer negativen Rotation um die Längsrichtung, kein Abtrennen von Knochenmaterial bewirkt. Durch das Vorsehen von unterschiedlichen Bearbeitungsstrukturen, nämlich Verdichtungsbearbeitungsstrukturen und Bohrbearbeitungsstrukturen, kann der Bearbeitungsbereich und dessen Bearbeitungsstrukturen in einfacher Weise hergestellt werden und zum einen eine Verdichtung und eine Knochenabtragung bewirken. Vorteilhafterweise ist zumindest eine Verdichtungsbearbeitungsstruktur, bevorzugt alle Verdichtungsbearbeitungsstrukturen, durch eine Freifläche gebildet und/oder wobei die Verdichtungsbearbeitungsstruktur oder - Verdichtungsbearbeitungsstrukturen einen Winkel in einem Bereich von 10° bis 45°, bevorzugt in einem Bereich von 12° bis 40°, mit einer Tangente in einer Schnittebene senkrecht zu der Längsrichtung ausbildet bzw. ausbilden. Die maßgebliche Tangente ist dabei diejenige Tangente an der bzw. einer Umhüllenden des Bearbeitungsbereich, an welchen der - wenn auch gedachte verlängerte - Verlauf der Verdichtungsbearbeitungsstruktur die Umhüllende des Bearbeitungsbereichs des Implantatbohrers schneidet. Die Umhüllende des Implantatbohrers bzw. des Bearbeitungsbereichs ist dabei der kleinstmögliche Kreis, dessen Mittelpunkt auf der Längsrichtung liegt und welcher in der Schnittebene gerade den Implantatbohrer bzw. den Bearbeitungsbereich umgeben kann. In anderen Worten kann die Umhüllende daher auch durch einen Kreis gebildet sein, der in einer Schnittebene liegt, welche senkrecht zu der Längsrichtung steht und wobei der Kreis gerade der kleinste mögliche Kreis ist, der den Bearbeitungsbereich in der Schnittebene umgeben kann. Sollte der Winkel der Verdichtungsbearbeitungsstruktur dabei in einem Bereich von 10° bis 45° liegen, so kann hierdurch eine besonders leicht zu fertigende Verdichtungsbearbeitungsstruktur erreicht werden. Sollte jedoch der Winkel in einem Bereich von 12° bis 40° liegen, so kann hierdurch eine besonders gute Knochenverdichtungsmöglichkeit erreicht werden, welche dennoch ein Überhitzen des Knochenmaterials während der Verdichtung verhindert und/oder dessen Auftretenswahrscheinlichkeit zumindest stark reduziert.

Zweckmäßigerweise ist die Verdichtungsbearbeitungsstruktur hinterschneidungsfrei mit den direkt benachbarten Strukturen, insbesondere in einer Schnittebene senkrecht zu der Längsrichtung, ausgebildet. Durch diese hinterschneidungsfreie Ausbildung mit benachbarten Strukturen kann insbesondere ein Abtrennen von Knochenmaterial während einer Rotation um die Längsrichtung durch die Verdichtungsbearbeitungsstruktur oder die Verdichtungsbearbeitungsstrukturen verhindert werden.

Vorteilhafterweise ist der Winkel der Verdichtungsbearbeitungsstruktur entlang des Verlaufs in Längsrichtung variabel. In anderen Worten kann daher der Winkel, den die Verdichtungsbearbeitungsstruktur in unterschiedlichen Schnittebenen entlang der Längsrichtung ausbildet, unterschiedlich sein. Hierdurch kann insbesondere die Verdichtungswirkung der Verdichtungsbearbeitungsstruktur an den Außendurchmesser des Bohrers angepasst werden. Dies ist insbesondere aufgrund des Umstandes vorteilhaft, dass unterschiedliche Durchmesser unterschiedliche Anstellungswinkel für eine Knochenverdichtung verlangen. Zweckmäßigerweise ist dabei der Winkel der Verdichtungsbearbeitungsstrukturen entlang des Verlaufs in Längsrichtung jedoch in den jeweiligen Bearbeitungsabschnitten konstant. Hierdurch kann die Fertigung des Bohrers vereinfacht werden. In anderen Worten kann der Winkel z.B. in dem Hauptbearbeitungsabschnitt und/oder in jedem anderen Bearbeitungsabschnitt für sich jeweils konstant sein. Der Winkel kann sich jedoch jeweils am Übergang von einem in den anderen Bearbeitungsabschnitt ändern, sodass dieser "global" nicht konstant ist sondern variabel, insbesondere entlang der Längsrichtung.

Vorteilhafterweise liegt der Winkel der Verdichtungsbearbeitungsstruktur, bevorzugt aller Verdichtungsbearbeitungsstrukturen, in dem Axialbearbeitungsabschnitt und/oder in dem Zylinderbearbeitungsabschnitt in einem Bereich von 12° bis 18°. Hierdurch kann eine besonders gute Verdichtungswirkung bei kleinen Durchmessern des Bohrers erreicht werden, wobei dennoch eine Überhitzung des Knochenmaterials vermieden bzw. zumindest stark reduziert werden kann.

Vorteilhafterweise liegt der Winkel der Verdichtungsbearbeitungsstruktur, bevorzugt aller Verdichtungsbearbeitungsstrukturen, in dem Hauptbearbeitungsabschnitt und/oder in dem Übergangsbearbeitungsabschnitt in einem Bereich von 30° bis 40°. Gerade bei großflächigen Bearbeitungsflächen bzw. Bearbeitungsstrukturen, welche vornehmlich keine axiale Bearbeitung bewirken, hat ein Winkel in einem Bereich von 30 bis 40°, besonders bevorzugt in einem Bereich von 33° bis 37°, eine besonders gute Verdichtungswirkung, wobei jedoch gleichzeitig eine Knochenüberhitzung vermieden werden kann und/oder zumindest reduziert werden kann.

Vorteilhafterweise ist der Winkel der Verdichtungsbearbeitungsstruktur, bevorzugt aller Verdichtungsbearbeitungsstrukturen, überwiegend konstant. Unter einem überwiegend konstant ist dabei zu verstehen, dass der Winkel nur in geringen Abschnitten in Längsrichtung gesehen verändert ist. In anderen Worten ist der Winkel der Verdichtungsbearbeitungsstruktur entlang der Längsrichtung gesehen zwar möglicherweise variabel, wobei die Summe der konstanten Abschnitte jedoch 70%, bevorzugt jedoch zumindest 80% und besonders bevorzugt zumindest 90% der Länge der Verdichtungsbearbeitungsstruktur in Längsrichtung ausbilden, um als überwiegend konstant im Sinne der Erfindung angesehen werden zu können. Beispielsweise kann - bis auf einen scharfen Übergang, der Winkel in dem Axialbearbeitungsabschnitt und oder in dem Zylinderbearbeitungsabschnitt einen ersten Wert einnehmen und in dem Hauptbearbeitungsabschnitt und/oder in dem Übergangsbearbeitungsabschnitt einen zweiten Wert annehmen. Diese unterschiedlichen Winkelbereiche werden dabei insbesondere durch einen scharfen Übergangsbereich begrenzt bzw. dieser scharfe Übergangsbereich selber weist einen variablen Winkel auf. Besonders bevorzugt kann der scharfe Übergangsbereich dabei als eine Kante ausgebildet sein, so dass ein sprunghafter Übergang resultiert. Alternativ bevorzugt kann der Übergangsbereich sich auch in einem Längenbereich von 0,01 bis 0,4 mm, bevorzugt in einem Bereich von 0,02 bis 0,1 mm, in Längsrichtung erstrecken. In anderen Worten kann bis auf den Übergangsbereich der Winkel jeweils abschnittsweise konstant sein.

Bevorzugt sind die Spiralnuten, bevorzugt alle Spiralnuten, in Umfangsrichtung durch eine Verdichtungsbearbeitungsstruktur und/oder durch eine Bohrbearbeitungsstruktur und/oder durch eine Übergangsfase begrenzt. In anderen Worten kann die Spiralnut, insbesondere in Umfangsrichtung um die Längsrichtung in positive und/oder negative Umfangsrichtung um die Längsrichtung durch jeweils eine Verdichtungsbearbeitungsstruktur, eine Bohrbearbeitungsstruktur und/oder eine Übergangsfase begrenzt sein. Hierdurch kann eine geringe Baugröße des Implantatbohrers erreicht werden. Eine Übergangsfase ist eine kurze Fase, insbesondere mit einer Kantenlänge im Bereich von 0,02 bis 0,1 mm, welche einen Übergang zu einer Verdichtungsbearbeitungsstruktur bereitstellt. Durch diese Übergangsfase kann insbesondere eine Hinterschneidung vermieden werden, so dass sichergestellt werden kann, dass die Verdichtungsbearbeitungsstruktur und der sie umgebende Bereich keine knochenabtragende Wirkung entfaltet.

Zweckmäßigerweise erstreckt sich zumindest eine Spiralnut, bevorzugt alle Spiralnuten, durch den Hauptbearbeitungsabschnitt, den Übergangsbearbeitungsabschnitt, den Zylinderbearbeitungsabschnitt und/oder durch den Axialbearbeitungsabschnitt. Durch das Ausbilden der Spiralnut derart, dass diese mehrere Bearbeitungsabschnitte, insbesondere alle Bearbeitungsabschnitte, in Längsrichtung durchläuft und/oder sich in diese Bereiche erstreckt, kann eine besonders einfache Fertigung des Implantatbohrers erreicht werden. Sollte die Spiralnut sich durch bzw. in alle Bereiche des Bearbeitungsabschnitts erstrecken, kann eine besonders gute Möglichkeit geschaffen werden, dass der Bohrer nicht nur bei der spanenden Bearbeitung sondern auch bei der verdichtenden Bearbeitung alle Bereiche des Lochs bzw. der Implantatausnehmung sicher (er)schaffen kann und wobei gleichzeitig auch die Spiralnut eine Kühlwirkung für das Knochenmaterial bereitstellen kann.

Vorteilhafterweise ist der Bearbeitungsbereich in Längsrichtung durch einen, insbesondere in Längsrichtung umlaufenden, Anschlagsflansch begrenzt. Hierdurch kann sichergestellt werden, dass ein zu tiefes Eindringen des Implantatbohrers in das Knochenmaterial formschlüssig verhindert ist. Daher kann durch einen derartigen Anschlagsflansch die Sicherheit des Implantatbohrers gesteigert werden.

Vorteilhafterweise ist der Implantatbohrer aus Titan oder einer Titanlegierung. Durch das Verwenden von Titan kann eine besonders gute Standfestigkeit des Bohrers und darüber hinaus auch eine besonders hohe mechanische Belastbarkeit des Bohrers erreicht werden, so dass dessen Sicherheit gesteigert werden kann und die Benutzungsdauer des Implantatbohrers positiv beeinflusst werden kann.

Zweckmäßigerweise ist der Implantatbohrer einstückig ausgebildet. Unter "einstückig" ist dabei insbesondere zu verstehen, dass das maßgebliche Bauteil grundlegend aus einem Körper hergestellt ist, welcher in einem einzigen Urformprozess geschaffen wurde. In anderen Worten ist daher eine Einstückigkeit dann nicht mehr gegeben, wenn verschiedene Bestandteile gefügt werden müssen, um einen Ausgangskörper bzw. Rohling zu erschaffen, aus welchem der Implantatbohrer geschaffen wurde. Durch die einstückige Ausgestaltung des Implantatbohrers kann eine besonders gute Festigkeit des Bohrers erreicht werden, so dass dieser, insbesondere in Hinblick auf eine dynamische und/oder schwellende Belastung, besonders vorteilhaft ausgebildet ist.

Vorteilhafterweise ist der Implantatbohrer als Massivteil ausgebildet. Unter einem Massivteil ist insbesondere zu verstehen, dass der Implantatbohrer nicht hohl ist.

In anderen Worten kann der Implantatbohrer ein Vollteil sein, welcher keine Bohrungen oder Durchbrüche aufweist. Hierdurch kann insbesondere die mechanische Stabilität positiv beeinflusst werden.

Nachfolgend wird eine nicht erschöpfende Aufzählung an möglichen vorteilhaften Ausführungsformen ausgeführt:
Ausführungsbeispiel 1:
   Implantatbohrer (1), insbesondere Dentalimplantatbohrer, umfassend einen Montagebereich (10) und einen Bearbeitungsbereich (30),
   wobei sich der Implantatbohrer (1) in eine Längsrichtung (L) erstreckt,
   wobei die distalen Endbereiche in Längsrichtung (L) des Implantatbohrers (1) insbesondere durch den Montagebereich (10) und den Bearbeitungsbereich (30) ausgebildet sind,
   wobei der Bearbeitungsbereich (30) zumindest zwei Bearbeitungsstrukturen (32) aufweist,
   wobei der Bearbeitungsbereich (30), insbesondere durch die Bearbeitungsstrukturen (32), derart ausgebildet ist,
   dass dieser bei einer positiven Rotation um die Längsrichtung (L) Knochenmaterial abtragen kann und
   dass dieser bei einer negativen Rotation um die Längsrichtung (L) Knochenmaterial verdichten kann,
   wobei der Bearbeitungsbereich (30) zumindest eine Verdichtungsbearbeitungsstruktur (50) und eine Bohrbearbeitungsstruktur (52) aufweist,
   wobei eine Umhüllende (70) durch einen Kreis gebildet ist, der in einer Schnittebene liegt, welche senkrecht zu der Längsrichtung (L) steht,
   wobei der Kreis (70) gerade der kleinste mögliche Kreis ist, der den Bearbeitungsbereich in der Schnittebene umgeben kann,
   wobei der zumindest "gedachte" Verlauf der Verdichtungsbearbeitungsstruktur (50) die Umhüllende (70) in der Schnittebene schneidet und die an diesen Schnittpunkt an die Umhüllende (70) angelegte Tangente (72) einen Winkel (W1) mit der Verdichtungsbearbeitungsstruktur (50) bzw. dem gedachten Verlauf der Verdichtungsbearbeitungsstruktur (50) aufweist,
   wobei der Winkel (W1) in einem Bereich von 10° bis 45°, bevorzugt in einem Bereich von 12° bis 40°, liegt,
   dadurch gekennzeichnet, dass der Bearbeitungsbereich (30) umlaufende Kühlnuten (36) aufweist.
Ausführungsbeispiel 2:
   Implantatbohrer (1) gemäß Ausführungsbeispiel 1,
   wobei der Bearbeitungsbereich (30) zumindest zwei, bevorzugt zumindest drei, Spiralnuten (34) aufweist,
   wobei die Spiralnuten (34) zumindest teilweise die Bearbeitungsstrukturen (32) oder einige Bearbeitungsstrukturen (32) ausbilden und/oder begrenzen.
Ausführungsbeispiel 3:
   Implantatbohrer (1) gemäß einem der vorhergehenden Ausführungsbeispiele,
   wobei der Bearbeitungsbereich (30) entlang der Längsrichtung (L) unterschiedliche Bearbeitungsabschnitte (B1, B2, B3, B4) aufweist,
   wobei der Implantatbohrer (1) vorzugsweise zumindest drei Bearbeitungsabschnitte (B1, B2, B3, B4) aufweist,
   wobei die Bearbeitungsabschnitte (B1, B2, B3, B4) unterschiedliche Außenkontursteigungswinkel (α1, α3) mit der Längsrichtung (L) ausbilden oder zylindrisch um die Längsrichtung (L) ausgebildet sind.
Ausführungsbeispiel 4:
   Implantatbohrer (1) gemäß einem der vorhergehenden Ausführungsbeispiele,
   wobei der Implantatbohrer (1), insbesondere der Bearbeitungsbereich (30), durch einen Axialbearbeitungsabschnitt (B1) in Längsrichtung (L) begrenzt ist,
   wobei der Axialbearbeitungsabschnitt (B1) einen Außenkontursteigungswinkel (α1) mit der Längsrichtung (L) ausbildet,
   wobei der Außenkontursteigungswinkel (α1) des Axialbearbeitungsabschnitts (B1) in einem Bereich von 60° bis 87° liegt und/oder
   wobei die Länge des Axialbearbeitungsabschnitt (B1) in Längsrichtung (L) in einem Bereich von 0,2 mm bis 0,4 mm liegt.
Ausführungsbeispiel 5:
   Implantatbohrer (1) gemäß einem der vorhergehenden Ausführungsbeispiele wobei die Kühlnuten (36) insbesondere geschlossene Ringe um die Längsrichtung (L) ausbilden.
Ausführungsbeispiel 6:
   Implantatbohrer (1) gemäß einem der vorhergehenden Ausführungsbeispiele,
   wobei der Implantatbohrer (1), insbesondere der Bearbeitungsbereich (30), einen Zylinderbearbeitungsabschnitt (B2) aufweist,
   wobei der Zylinderbearbeitungsabschnitt (B2) zylindrisch um die Längsrichtung (L) ausgebildet ist.
Ausführungsbeispiel 7:
   Implantatbohrer (1) gemäß einem der vorhergehenden Ausführungsbeispiele,
   wobei der Implantatbohrer (1), insbesondere der Bearbeitungsbereich (30), einen Übergangsbearbeitungsabschnitt (B3) aufweist,
   wobei der Übergangsbearbeitungsabschnitt (B3) insbesondere einen Außenkontursteigungswinkel (α3) mit der Längsrichtung (L) ausbildet,
   wobei der Außenkontursteigungswinkel (α3) des Übergangsbearbeitungsabschnitts (B3) in einem Bereich von 7° bis 15° liegen kann.
Ausführungsbeispiel 8:
   Implantatbohrer (1) gemäß einem der vorhergehenden Ausführungsbeispiele,
   wobei zumindest eine Verdichtungsbearbeitungsstruktur (50), bevorzugt alle Verdichtungsbearbeitungsstrukturen (50), durch eine Freifläche gebildet ist oder sind.
Ausführungsbeispiel 9:
   Implantatbohrer (1) gemäß einem der vorhergehenden Ausführungsbeispiele,
   wobei der Winkel (W1) der Verdichtungsbearbeitungsstruktur (50) entlang des Verlaufs in Längsrichtung (L) variabel ist.
Ausführungsbeispiel 10:
   Implantatbohrer (1) gemäß einem der vorhergehenden Ausführungsbeispiele,
   wobei die Spiralnuten (34), bevorzugt alle Spiralnuten (34), in Umfangsrichtung durch eine Verdichtungsbearbeitungsstruktur (50) und/oder durch eine Bohrbearbeitungsstruktur (52) oder eine Übergangsfase (54) begrenzt ist.
Ausführungsbeispiel 11:
   Implantatbohrer (1) gemäß einem der vorhergehenden Ausführungsbeispiele,
   wobei sich zumindest eine Spiralnut (34), bevorzugt alle Spiralnuten (34), durch den Hauptbearbeitungsabschnitt (B4), den Übergangsbearbeitungsabschnitt (B3), den Zylinderbearbeitungsabschnitt (B2) und/oder durch den Axialbearbeitungsabschnitt (B1) erstreckt.
Ausführungsbeispiel 12:
   Implantatbohrer (1) gemäß einem der vorhergehenden Ausführungsbeispiele,
   wobei der Bearbeitungsbereich (30) in Längsrichtung (L) durch einen, insbesondere um die Längsrichtung (L) umlaufenden, Anschlagsflansch (60) begrenzt ist.
Ausführungsbeispiel 13:
   Implantatbohrer (1) gemäß einem der vorhergehenden Ausführungsbeispiele,
   wobei der Implantatbohrer (1) einstückig ist.
Ausführungsbeispiel 14:
   Implantatbohrer (1) gemäß einem der vorhergehenden Ausführungsbeispiele,
   wobei der Implantatbohrer (1) als Massivteil ausgebildet ist.

Weitere Vorteile und Merkmale der vorliegenden Erfindung ergeben sich aus der nachfolgenden Beschreibung mit Bezug auf die Figuren. Einzelne Merkmale der dargestellten Ausführungsformen können dabei auch in anderen Ausführungsformen eingesetzt werden, sofern dies nicht ausdrücklich ausgeschlossen wurde. Es zeigen:
- Figur 1: eine Seitenansicht eines Implantatbohrers;
- Figur 2: eine Detailansicht eines Implantatbohrers im Bereich des Bearbeitungsbereichs;
- Figur 3: einen Schnitt durch einen Bearbeitungsbereich des Implantatbohrers;
- Figur 4: einen weiteren Schnitt durch den Bearbeitungsbereich eines Implantatbohrers;
- Figur 5: eine isometrische Ansicht eines Implantatbohrers;
- Figur 6: einen Implantatbohrerrohling in einer Seitenansicht;
- Figur 7: eine Axialansicht eines Implantatbohrers; und
- Figur 8: eine Prinzipansicht eines Schnittes eines Bearbeitungsbereichs des Implantatbohrers mit einer Umhüllenden.

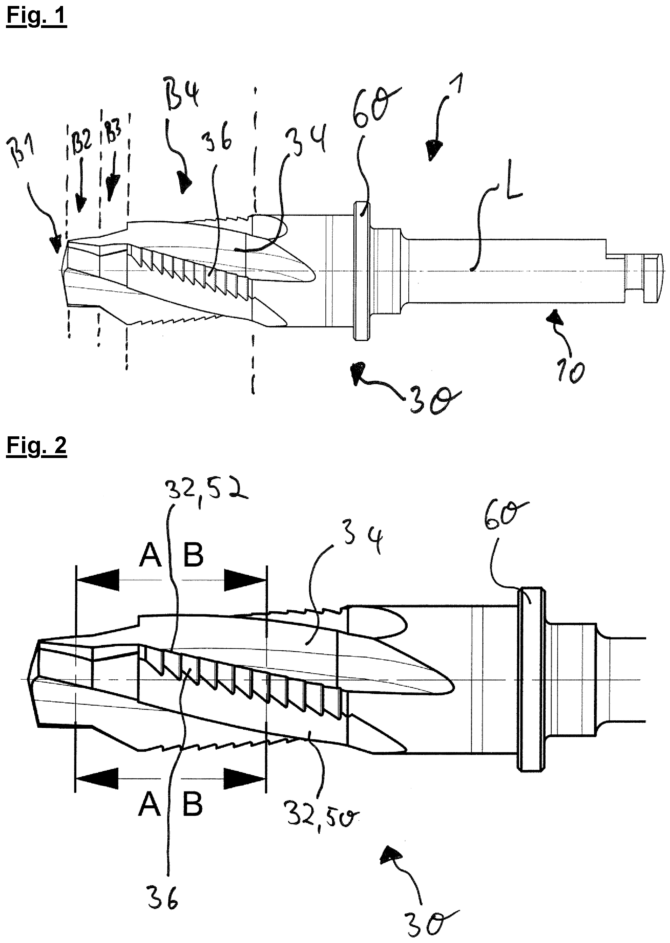

In **Figur 1** ist eine Seitenansicht eines Implantatbohrers 1 gezeigt. Die distalen Enden in Längsrichtung L des Implantatbohrers 1 sind dabei durch den Montagebereich 10 und durch den Bearbeitungsbereich 30 gebildet. In axialer Richtung bzw. in Längsrichtung L ist der Bearbeitungsbereich 30 durch den Axialbearbeitungsabschnitt B1 und durch den Anschlagsflansch 60 begrenzt. Der Bearbeitungsbereich 30 kann in vier Abschnitte zerlegt werden bzw. wird durch diese Bereiche ausgebildet, welche in Längsrichtung nebeneinander angeordnet sind, wobei diese Bearbeitungsabschnitte der Axialbearbeitungsabschnitt B1, der Zylinderbearbeitungsabschnitt B2, der Übergangsbearbeitungsabschnitt B3 und der Hauptbearbeitungsabschnitt B4 sind. Der Bearbeitungsbereich 30 weist eine Vielzahl von Spiralnuten 34 auf, welche sich spiralförmig um die Längsrichtung L erstrecken und sich vom Axialbearbeitungsabschnitt B1, über den Zylinderbearbeitungsabschnitt B2, über den Übergangsbearbeitungsabschnitt B3 und in den Hauptbearbeitungsabschnitt B4 erstrecken. Um eine besonders gute Kühlwirkung während der Bearbeitung eines Knochens zu erreichen, weist der Hauptbearbeitungsabschnitt B4 eine Vielzahl von Kühlnuten 36 auf, welche ringförmig um die Längsrichtung L ausgebildet sind. Um eine Festlegung des Implantatbohrers 1 in einem Bohrer bzw. einem Handwerkzeug zu erreichen, verfügt der Montagebereich 10 über eine Abflachung und eine umlaufende Nut, um so eine formschlüssige Drehmomentübertragung um die Längsrichtung auf den Implantatbohrer 1 und eine Axialkraftübertragung in Richtung der Längsrichtung L formschlüssig erreichen zu können. Der Hauptbearbeitungsabschnitt B4 kann sich dabei bis zu dem Anschlagsflansch 60 in Längsrichtung L erstrecken.

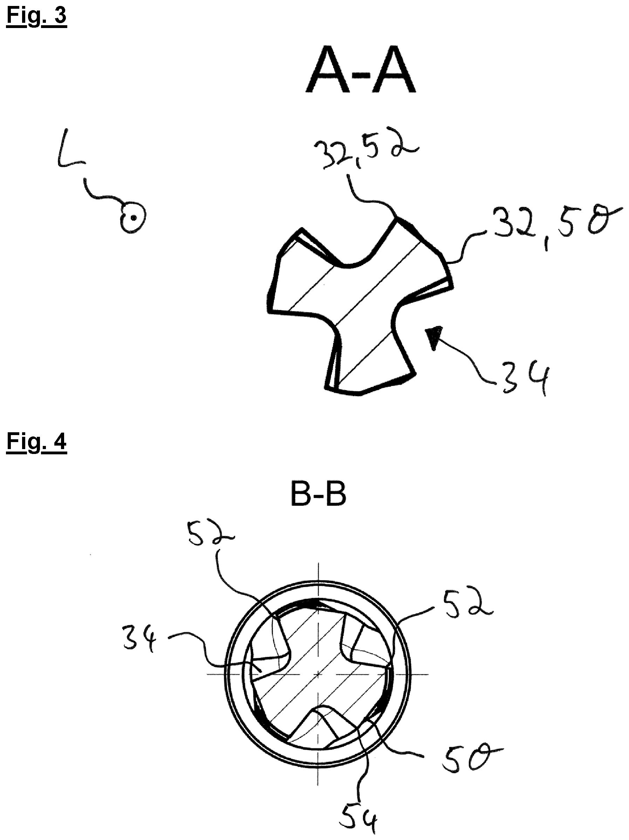

In **Figur 2** ist eine Detailansicht eines Bearbeitungsbereichs 30 dargestellt. Der in der Figur 2 gezeigte Bearbeitungsbereich 30 kann dabei insbesondere dem in der Figur 1 gezeigten Bearbeitungsbereich 30 entsprechen. In der Figur 2 sind zwei mit Pfeilen gekennzeichnete Schnittebenen angegeben, welche mit A und B gekennzeichnet sind. Mögliche Ausgestaltungen dieser Schnittebenen finden sich für die Schnittebene A-A in der Figur 3 und B-B in der Figur 4. Der Bearbeitungsbereich 30 weist eine Vielzahl von Spiralnuten 34 auf. Diese Spiralnuten 34 sind in Umfangsrichtung um die Längsrichtung L durch Bearbeitungsstrukturen 32 begrenzt. Einseitig in Umfangsrichtung ist die Spiralnut 34 dabei durch Bohrbearbeitungsstrukturen 52 begrenzt und in die entgegengesetzte Umfangsrichtung durch Verdichtungsbearbeitungsstrukturen 50. Die Verdichtungsbearbeitungsstrukturen 50 können jedoch über eine Übergangsfase 54 von der Spiralnut 34 beabstandet sein. In anderen Worten kann eine kurze Übergangsfase 54 ausgangsseitig an der Spiralnut 34 vorgesehen sein, in welche sich dann die Verdichtungsbearbeitungsstruktur 50 in Umfangsrichtung gesehen angliedert. Der axiale Auslauf der Spiralnut 34 befindet sich dabei in dem Hauptbearbeitungsabschnitt B4 in Längsrichtung L gesehen.

In **Figur 3** ist eine mögliche Schnittansicht der in der Figur 2 mit A-A gekennzeichneten Schnittebene dargestellt. Wie der Figur 3 entnommen werden kann, weist der Implantatbohrer 1 drei äquidistant in Umfangrichtung angeordnete Spiralnuten 34 auf. Die Spiralnuten 34 sind dabei am äußeren Schnittpunkt mit der Umhüllenden derart ausgebildet, dass diese entweder eine Verdichtungsbearbeitungsstruktur 50 oder eine Bohrbearbeitungsstruktur 52 ausbilden. Diese beiden Arten der Bearbeitungsstrukturen 32 dienen dabei dazu, eine Ausnehmung bzw. eine Bohrung in ein Knochenmaterial einzubringen, um so die Platzierung eines Implantats innerhalb dieses Knochenmaterials zu ermöglichen.

In **Figur 4** ist eine mögliche Ausgestaltung des in der Figur 2 mit B-B dargestellten Schnittes gezeigt. Der Schnitt B-B verläuft dabei vorzugsweise durch den Hauptbearbeitungsabschnitt B4. Auch im Hauptbearbeitungsabschnitt B4 sind die Spiralnuten 34 durch Bohrbearbeitungsstrukturen 32 und/oder Verdichtungsbearbeitungsstrukturen 40 und/oder Übergangsphasen 54 in eine Umfangsrichtung um die Längsrichtung L begrenzt. In anderen Worten können die distalen Endbereiche der Spiralnut 34 die Bearbeitungsstrukturen 32 und/oder eine Übergangsphase 54 ausbilden.

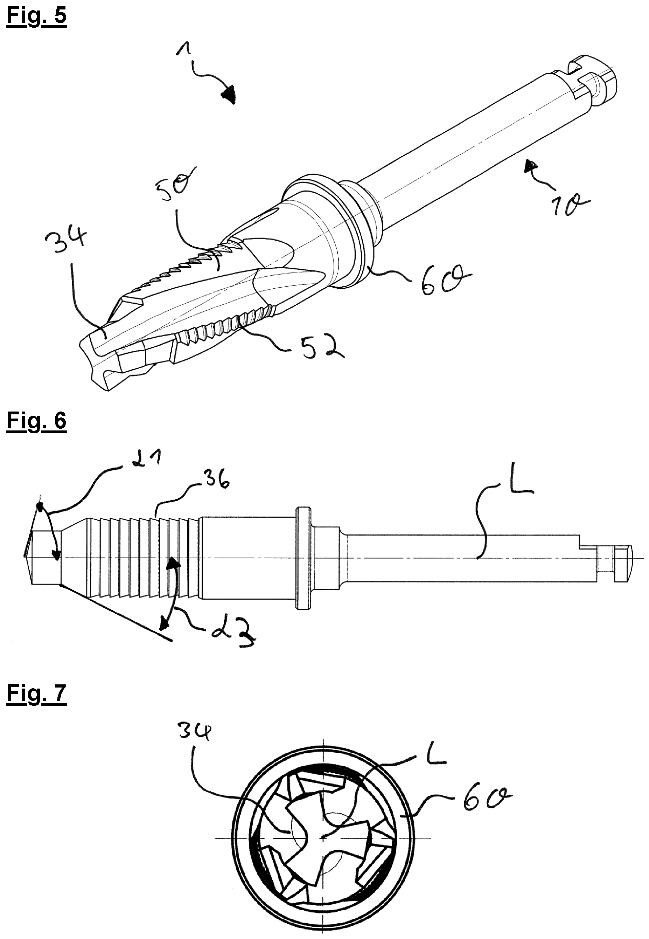

In **Figur 5** ist eine erläuternde isometrische Ansicht eines Implantatbohrers 1 gezeigt. In der Figur 5 ist besonders gut die abgeflachte Drehmomentübertragungsfläche im Montagebereich 10 des Implantatbohrers 1 zu erkennen. Wie der Figur 5 entnommen werden kann, erstrecken sich die Spiralnuten 34 dabei von einem axialen Ende in Längsrichtung L des Bearbeitungsbereichs 30 über weite Teile des Bearbeitungsbereichs 30.

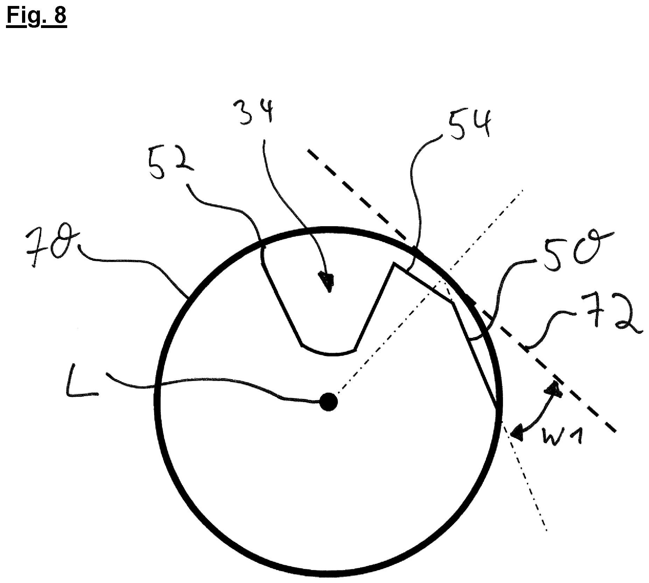

In **Figur 6** ist ein Rohling bzw. ein Halbzeug eines Implantatbohrers 1 gezeigt. Erläuternd in der Figur 6 ist dabei der Außenkontursteigungswinkel α1 des Axialbearbeitungsabschnitts B1 und der Außenkontursteigungswinkel α3 des Übergangsbearbeitungsabschnitts B3 zu erkennen. In dem in der Figur 6 gezeigten Implantatbohrer 1 bzw. dem Implantatbohrerrohling sind keine Spiralnuten 34 eingebracht, so dass der Implantatbohrer 1 bzw. der Implantatbohrerrohling - wie er in der Figur 6 gezeigt ist - ohne Bearbeitungsstrukturen 32 ausgebildet ist. Wie jedoch der Figur 6 zu entnehmen ist, sind die Kühlnuten 36 bereits in den Bearbeitungsabschnitt 30 des Rohlings eingebracht, welche kreisringförmig um die Längsrichtung L in geschlossenen Ringen ausgebildet sind. Wie der Figur 6 - im Vergleich zu der Figur 1 - zu entnehmen ist, sind dabei der Zylinderbearbeitungsabschnitt B2 und der Hauptbearbeitungsabschnitt B4 zylindrisch um die Längsrichtung L ausgebildet. Prinzipiell kann der in der Figur 6 gezeigte Implantatbohrer 1 bzw. der Implantatbohrerrohling dazu genutzt werden, den in den Figuren 1, 2, 3, 4, 5 oder 7 als auch prinzipiell dem in der Figur 8 dargestellten Ausführungsbeispiel als Ausgangswerksstück zu dienen. In anderen Worten kann aus dem Rohling gemäß der Figur 6 ein Implantatbohrer gemäß den Figuren 1 bis 5 und 7 bis 8 ausgebildet werden.

In **Figur 7** ist eine Ansicht eines Implantatbohrers 1 in Richtung der Längsrichtung L gezeigt. Die positive Längsrichtung L kann dabei von dem Montagebereich 10 zum Bearbeitungsbereich 30 weisen oder vom Bearbeitungsbereich 30 zum Montagebereich 10. Vorteilhafterweise ist der Implantatbohrer 1 derart ausgebildet, dass dieser bei einer Rechtsdrehung eine bohrende Bearbeitung erreicht und bei einer Linksdrehung eine verdichtende Bearbeitung. Eine derartige Ausgestaltung kann dabei beispielsweise durch die in der Figur 7 dargestellte Ausgestaltung erreicht werden.

In **Figur 8** ist eine prinzipiell erläuternde, wenn auch nur schematische, Darstellung gezeigt, um eine Ausgestaltung einer Verdichtungsbearbeitungsstruktur 50 erläuternd zu zeigen. In der Figur 8 ist dabei eine Schnittebene senkrecht zur Längsrichtung L gezeigt, wobei sich diese Schnittebene innerhalb des Bearbeitungsbereichs 30 befindet. Die Schnittebene in Längsrichtung L durchstoßend ist eine Spiralnut 34 in dem Implantatbohrer 1 vorgesehen, welche in Umfangsrichtung entgegen dem Uhrzeigersinn durch eine Bohrbearbeitungsstruktur 52 begrenzt ist. Im Uhrzeigersinn in Umfangsrichtung schließt sich an die Spiralnut 34 eine Übergangsfase 54 an, an welche sich im Uhrzeigersinn in Umfangsrichtung eine Verdichtungsbearbeitungsstruktur 50 anschließt. Der Bearbeitungsbereich 30 ist dabei durch die Umhüllende 70 umhüllt, wobei der Mittelpunkt dieser "gedachten" Umhüllenden 70 auf der Längsrichtung L liegt. Der zumindest "gedachte" Verlauf der Verdichtungsbearbeitungsstruktur 50 schneidet dabei die Umhüllende 70 in der dargestellten Schnittebene. Die an diesen Schnittpunkt an die Umhüllende 70 angelegte Tangente 72 weist einen Winkel W1 mit der Verdichtungsbearbeitungsstruktur 50 bzw. dem gedachten Verlauf der Verdichtungsbearbeitungsstruktur 50 auf.

### Bezugszeichenliste:

- 1: - Implantatbohrer
- 10: - Montagebereich
- 30: - Bearbeitungsbereich
- 32: - Bearbeitungsstrukturen
- 34: - Spiralnut
- 36: - Kühlnut
- 50: - Verdichtungsbearbeitungsstruktur
- 52: - Bohrbearbeitungsstruktur
- 54: - Übergangsfase
- 60: - Anschlagsflansch
- 70: - Umhüllende
- 72: - Tangente
- B1: - Axialbearbeitungsabschnitt
- B2: - Zylinderbearbeitungsabschnitt
- B3: - Übergangsbearbeitungsabschnitt
- B4: - Hauptbearbeitungsabschnitt
- L: - Längsrichtung
- W1: - Winkel der Verdichtungsbearbeitungsstruktur
- α1: - Außenkontursteigungswinkel des Axialbearbeitungsabschnitts
- α3: - Außenkontursteigungswinkel des Übergangsbearbeitungsabschnitts

## Patentansprüche

1. Implantatbohrer (1), insbesondere Dentalimplantatbohrer, umfassend einen Montagebereich (10) und einen Bearbeitungsbereich (30),
wobei sich der Implantatbohrer (1) in eine Längsrichtung (L) erstreckt,
wobei die distalen Endbereiche in Längsrichtung (L) des Implantatbohrers (1) insbesondere durch den Montagebereich (10) und den Bearbeitungsbereich (30) ausgebildet sind,
wobei der Bearbeitungsbereich (30) zumindest zwei Bearbeitungsstrukturen (32) aufweist,
wobei der Bearbeitungsbereich (30), insbesondere durch die Bearbeitungsstrukturen (32), derart ausgebildet ist,
dass dieser bei einer positiven Rotation um die Längsrichtung (L) Knochenmaterial abtragen kann, und
dass dieser bei einer negativen Rotation um die Längsrichtung (L) Knochenmaterial verdichten kann.

2. Implantatbohrer (1) gemäß Anspruch 1,
wobei der Implantatbohrer (1) bzw. der Bearbeitungsbereich (30), insbesondere die Bearbeitungsstrukturen (32), derart ausgebildet ist, dass dieser bei einer positiven Rotation um die Längsrichtung (L) ausschließlich Knochenmaterial abtragen kann, insbesondere durch einen ausschließlichen Kontakt von geometrisch bestimmten Schneiden mit dem Knochenmaterial.

3. Implantatbohrer (1) gemäß einem der vorhergehenden Ansprüche,
wobei der Implantatbohrer (1) bzw. der Bearbeitungsbereich (30), insbesondere die Bearbeitungsstrukturen (32), derart ausgebildet ist,
dass dieser bei einer negativen Rotation um die Längsrichtung (L) ausschließlich Knochenmaterial verdichten kann, insbesondere indem ausschließlich knochenverdichtende Bearbeitungsstrukturen mit dem Knochen in Kontakt gelangen.

4. Implantatbohrer (1) gemäß einem der vorhergehenden Ansprüche,
wobei der Bearbeitungsbereich (30) eine Bohrbearbeitungsstruktur (52) aufweist, wobei die Bohrbearbeitungsstruktur (52) eine Schnittkante von zwei ineinander übergehenden Flächen ist, wobei diese Flächen eine Hinterschneidung miteinander ausbilden.

5. Implantatbohrer (1) gemäß einem der vorhergehenden Ansprüche,
wobei der Bearbeitungsbereich (30) zumindest eine Verdichtungsbearbeitungsstruktur (50) aufweist, wobei die Verdichtungsbearbeitungsstruktur (50) durch eine nachlaufende Fläche gebildet ist, die dazu ausgelegt ist, einen Druck auf den Knochen auszuüben, um diesen, insbesondere in Normalenrichtung zu der Wand des zu kreierenden Lochs, zu verdrängen und so eine Verdichtung des Knochenmaterials zu erreichen.

6. Implantatbohrer (1) gemäß einem der vorhergehenden Ansprüche,
wobei der Bearbeitungsbereich (30) zumindest eine Verdichtungsbearbeitungsstruktur (50) aufweist, wobei die Verdichtungsbearbeitungsstruktur (50) durch eine Freiformfläche bzw. eine Freifläche gebildet ist, welche eine in Umlaufrichtung ansteigende Fläche ist, welche bei einer negativen Rotation um die Längsrichtung (L) derart ausgebildet ist, dass die Freifläche einen zunehmenden Abstand zur Rotationsachse bzw. zur Längsrichtung ausbildet.

7. Implantatbohrer (1) gemäß einem der vorhergehenden Ansprüche,
wobei der Bearbeitungsbereich (30) zumindest eine Verdichtungsbearbeitungsstruktur (50) aufweist, wobei die zumindest eine Verdichtungsbearbeitungsstruktur (50), bevorzugt alle Verdichtungsbearbeitungsstrukturen (50), durch eine Freifläche gebildet ist oder sind,
und/oder
wobei die Verdichtungsbearbeitungsstruktur (50) einen Winkel (W1) in einem Bereich von 10° bis 45°, bevorzugt in einem Bereich von 12° bis 40°, mit einer Tangente in einer Schnittebene senkrecht zu der Längsrichtung (L) ausbildet.

8. Implantatbohrer (1) gemäß Anspruch 7,
wobei der Winkel (W1) der Verdichtungsbearbeitungsstruktur (50) entlang des Verlaufs in Längsrichtung (L) variabel ist.

9. Implantatbohrer (1) gemäß einem der vorhergehenden Ansprüche,
wobei der Bearbeitungsbereich (30) zumindest eine Verdichtungsbearbeitungsstruktur (50) aufweist, wobei die Verdichtungsbearbeitungsstruktur (50) hinterschneidungsfrei mit den direkt benachbarten Strukturen, insbesondere in einer Schnittebene senkrecht zu der Längsrichtung (L), ausgebildet ist.

10. Implantatbohrer (1) gemäß einem der vorhergehenden Ansprüche,
wobei der Bearbeitungsbereich (30) Spiralnuten (34) aufweist, welche in Umfangsrichtung durch eine Verdichtungsbearbeitungsstruktur (50) und/oder eine Bohrbearbeitungsstruktur (52) und/oder eine Übergangsfase (54) begrenzt sind.

11. Implantatbohrer (1) gemäß Anspruch 10, wobei die Übergangsfase (54) eine kurze Fase, insbesondere mit einer Kantenlänge im Bereich von 0,02 bis 0,1 mm, ist, welche einen Übergang zu einer Verdichtungsbearbeitungsstruktur (50) bereitstellt.

12. Implantatbohrer (1) gemäß einem der vorhergehenden Ansprüche,
wobei der Bearbeitungsbereich (30) zumindest zwei, bevorzugt zumindest drei Spiralnuten (34) aufweist,
wobei die Spiralnuten (34) zumindest teilweise die Bearbeitungsstrukturen (32) oder einige Bearbeitungsstrukturen (32) ausbilden und/oder begrenzen,
wobei sich zumindest eine Spiralnut (34), bevorzugt alle Spiralnuten (34),
durch einen Hauptbearbeitungsabschnitt (B4), einen Übergangsbearbeitungsabschnitt (B3), einen Zylinderbearbeitungsabschnitt (B2) und/oder durch einen Axialbearbeitungsabschnitt (B1) erstreckt.

13. Implantatbohrer (1) gemäß einem der vorhergehenden Ansprüche,
wobei der Implantatbohrer (1), insbesondere der Bearbeitungsbereich (30), durch einen Axialbearbeitungsabschnitt (B1) in Längsrichtung (L) begrenzt ist,
wobei der Axialbearbeitungsabschnitt (B1) einen Außenkontursteigungswinkel (α1) mit der Längsrichtung (L) ausbildet,
wobei der Außenkontursteigungswinkel (α1) des Axialbearbeitungsabschnitts (B1) in einem Bereich von 60° bis 87° liegt und/oder
wobei die Länge des Axialbearbeitungsabschnitt (B1) in Längsrichtung (L) in einem Bereich von 0,2 mm bis 0,4 mm liegt.

14. Implantatbohrer (1) gemäß einem der vorhergehenden Ansprüche,
wobei der Implantatbohrer (1), insbesondere der Bearbeitungsbereich (30), einen Zylinderbearbeitungsabschnitt (B2) aufweist,
wobei der Zylinderbearbeitungsabschnitt (B2) zylindrisch um die Längsrichtung (L) ausgebildet ist.

15. Implantatbohrer (1) gemäß einem der vorhergehenden Ansprüche,
wobei der Implantatbohrer (1), insbesondere der Bearbeitungsbereich (30), einen Übergangsbearbeitungsabschnitt (B3) aufweist,
wobei der Übergangsbearbeitungsabschnitt (B3) insbesondere einen Außenkontursteigungswinkel (α3) mit der Längsrichtung (L) ausbildet,
wobei der Außenkontursteigungswinkel (α3) des Übergangsbearbeitungsabschnitts (B3) in einem Bereich von 7° bis 15° liegen kann.
